# EUROPÄISCHE PATENTSCHRIFT

(11) **EP 1 949 878 B1**
(45) Veröffentlichungstag und Bekanntmachung des Hinweises auf die Patenterteilung: **02.11.2011**
(21) Anmeldenummer: 07023096.6
(22) Anmeldetag: 29.11.2007
(51) Int. Cl.: A61F 9/02, G02C 7/10, G02C 9/04

(54) **Brille, insbesondere Sportbrille**
Goggles, in particular sport goggles
Lunettes, en particulier lunettes de sport

(30) Priorität: 25.01.2007 DE 202007001104 U
(43) Veröffentlichungstag der Anmeldung: 30.07.2008
(73) Patentinhaber: ALPINA SPORTS GmbH, 86316 Friedberg (DE)
(72) Erfinder: Grau, Werner, 86316 Haberskirch (DE); Gehring, Martin, 86655 Harburg/Mündling (DE)
(74) Vertreter: Schneck, Herbert

(56) Entgegenhaltungen:
- WO-A-2006/035466
- US-A- 5 455 639
- US-A1- 2006 191 062

## Beschreibung

Die Erfindung richtet sich auf eine Brille, insbesondere eine Sportbrille oder Skibrille, mit einem Rahmen und einer am Rahmen angeordneten Scheibe sowie mit einer über dieser Scheibe lösbar befestigbaren Deckscheibe.

Derartige Deckscheiben ermöglichen es, über den Windschutz hinaus einen Schutz gegen Sonneneinstrahlung oder eine Kontrasterhöhung bei schlechten Sichtverhältnissen durch eine entsprechende Tönung zu realisieren.

Herkömmlicherweise werden sie auswechselbar dadurch an der Brille festgelegt, dass sie mittels eines druckknopfartigen Haltemechanismus angebracht werden.

US-A-2006/0191062 offenbart die technischen Merkmale des Oberbegriffs aus Anspruch 1.

Hiervon ausgehend liegt der Erfindung die Aufgabe zugrunde, eine solche Brille kostengünstig herstellbar zu machen, wobei die Handhabung beim Festlegen der Deckscheibe und bei deren Austausch erleichtert werden soll und die Deckscheibe im festgelegten Zustand exakt und zuverlässig gehalten ist.

Diese Aufgabe wird erfindungsgemäß dadurch gelöst, dass an jedem äußeren Ende der Deckscheibe ein Deckscheibenspanner mit einer Ausnehmung angeordnet ist und an jedem äußeren Ende des Rahmens ein Spannhaken ausgebildet ist, wobei die Deckscheibenspanner wenigstens abschnittsweise aus einem elastisch dehnbaren Material bestehen, wobei der Abstand der Spannhaken längs der Scheibe größer ist als der Abstand der hinteren Enden der Ausnehmungen, sodass die hinteren Enden der Ausnehmungen nur unter elastischer Dehnung der Deckscheibenspanner über die hinteren Enden der Spannhaken hinter die Spannhaken einrastbar sind. Vorteilhafterweise ist vorgesehen, dass anschließend an das hintere Ende der Ausnehmungen Handhabungsansätze vorgesehen sind, die im eingesetzten, eingespannten Zustand am hinteren Ende der Spannhaken anliegen.

Eine günstige Ausgestaltung sieht vor, dass die Deckscheibenspanner einen Grundkörper aus einem elastischen Material aufweisen, der mit einer umlaufenden Nut versehen ist, sodass der Grundkörper in eine Ausnehmung der Deckscheibe derart einsetzbar ist, dass der Rand der Ausnehmung der Deckscheibe in der Nut des Grundkörpers zu liegen kommt und der Deckscheibenspanner derart an der Deckscheibe fixiert ist.

Nachfolgend wird die Erfindung anhand eines bevorzugten Ausführungsbeispiels in Verbindung mit der Zeichnung näher erläutert. Dabei zeigen:

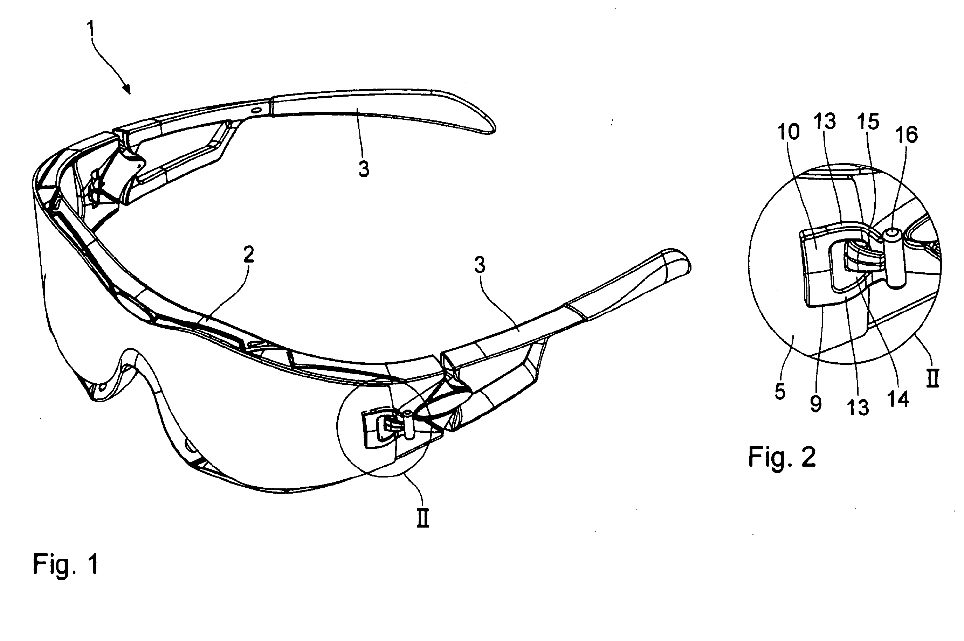

- Fig. 1: eine perspektivische Ansicht einer erfindungsgemäßen Brille von schräg oben,
- Fig. 2: eine vergrößerte Darstellung des Bereichs II in Fig. 1,
- Fig. 3: eine Ansicht der Brille von vorne und
- Fig. 4: einen Schnitt längs der Linie IV-IV in Fig. 3.

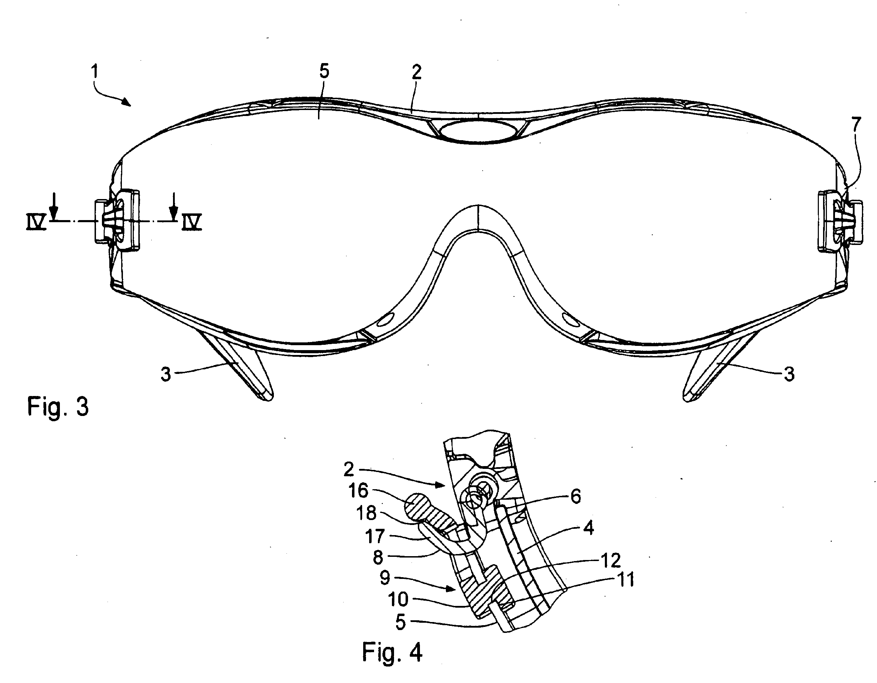

Eine in der Zeichnung dargestellte Brille 1 umfasst einen Rahmen 2, an dem Rahmen 2 angelenkte Bügel 3, eine durchgehende Scheibe 4 und eine Deckscheibe 5. Dabei ist die Scheibe 4, wie aus Fig. 4 ersichtlich, in einer Nut 6 des Rahmens 2 festgelegt. Die Deckscheibe 5 kann über der Scheibe 4 fakultativ befestigt werden und ist austauschbar.

Zur Befestigung der Deckscheibe 5 sind an seitlichen Rahmenteilen 7 des Rahmens 2 Spannhaken 8 ausgebildet, die eine annähernd U-förmige Querschnittskonfiguration aufweisen und sich nach hinten zu den Bügeln 3 hin öffnen, wobei die U-Konfiguration in einer - bezogen auf den Tragezustand - etwa horizontalen Ebene liegt.

An der Deckscheibe 5 sind Deckscheibenspanner 9 aus einem elastischen, dehnfähigen Material befestigt.

Die Deckscheibenspanner 9 weisen einen blockartigen Grundkörper 10 mit einer umlaufenden Nut 11 auf, sodass der elastische Grundkörper 10 in eine Ausnehmung 12 der Deckscheibe 5 derart einsetzbar ist, dass die Ränder der Ausnehmung 12 in der Nut 11 zu liegen kommen. Hierdurch sind die Deckscheibenspanner 9 festgelegt. Ausgehend von dem Grundkörper 10 erstrecken sich Schenkel 13 in Richtung auf die Bügel 3, welche eine Ausnehmung 14 umschließen. Am bügelseitigen Ende 15 der Schenkel 13 enden diese an einem vertikalen walzenartigen Handhabungsansatz 16, der es dem Benutzer ermöglicht, das geschlossene hintere Ende des Deckscheibenspanners 9 hinter dem jeweiligen Spannhaken zu positionieren, wobei das Ende 17 des Spannhakens 8 in eine Vertiefung 18 eingreift, die hinter dem Handhabungsansatz 16 ausgebildet ist, sodass eine sichere Positionierung gewährleistet ist.

Die Befestigung der Deckscheibe 5 erfolgt also so, dass zunächst der Deckscheibenspanner 9 an einer Seite in den dortigen Spannhaken 8 eingehängt wird.

Der Abstand der Ausnehmungen 14 bzw. der bügelseitigen Enden 15 der Ausnehmungen 14 der Deckenscheibenspanner 9 an der Deckscheibe 5 ist so dimensioniert, dass beim Herumführen der Deckscheibe 5 ausgehend von dem ersten Spannhaken 8 über die Scheibe 4 das hintere Ende der Ausnehmung 14 des gegenüberliegenden Deckscheibenspanners 9 vor dem Ende des gegenüberliegenden Spannhakens 8 zu liegen kommt. Erst wenn nun eine Zugspannung aufgebracht wird, werden die Deckscheibenspanner 9, insbesondere deren Schenkel 13 elastisch gedehnt und der Handhabungsansatz 16 des zweiten Deckscheibenspanners 9 kann hinter dem zweiten Spannhaken 8 einrasten.

Das Lösen der Deckscheibe 5 erfolgt in umgekehrter Richtung, indem der Handhabungsabschnitt 16 eines Endes der Deckscheibe 5 nach hinten gezogen und über den jeweiligen Spannhaken 8 gehoben wird.

## Patentansprüche

1. Brille, insbesondere Sportbrille oder Skibrille, mit einem Rahmen und einer am Rahmen angeordneten Scheibe sowie mit einer über dieser Scheibe lösbar befestigbaren Deckscheibe, **dadurch gekennzeichnet, dass** an jedem äußeren Ende (17) der Deckscheibe (5) ein Deckscheibenspanner (9) mit einer Ausnehmung (14) angeordnet ist und an jedem äußeren Ende des Rahmens (2) ein Spannhaken (8) ausgebildet ist, wobei die Deckscheibenspanner (9) wenigstens abschnittsweise aus einem elastisch dehnbaren Material bestehen, wobei der Abstand der Spannhaken (8) längs der Scheibe (4) größer ist als der Abstand der hinteren Enden der Ausnehmungen (14), sodass die hinteren Enden der Ausnehmungen (14) nur unter elastischer Dehnung der Deckscheibenspanner (9) über die hinteren Enden der Spannhaken (8) hinter die Spannhaken (8) einrastbar sind.

2. Brille nach Anspruch 1, **dadurch gekennzeichnet, dass** anschließend an das hintere Ende der Ausnehmungen (14) Handhabungsansätze (16) vorgesehen sind, die im eingesetzten, eingespannten Zustand am hinteren Ende der Spannhaken (8) anliegen.

3. Brille nach Anspruch 1, **dadurch gekennzeichnet, dass** die Deckscheibenspanner (9) einen Grundkörper (10) aus einem elastischen Material aufweisen, der mit einer umlaufenden Nut (11) versehen ist, sodass der Grundkörper (10) in eine Ausnehmung der Deckscheibe (5) derart einsetzbar ist, dass der Rand der Ausnehmung der Deckscheibe (5) in der Nut (11) des Grundkörpers (10) zu liegen kommt und der Deckscheibenspanner (8) derart an der Deckscheibe (5) fixiert ist.

## Claims

1. Goggles, in particular sports goggles or ski goggles, with a frame and a lens arranged on the frame and a cover lens which can be secured detachably over said lens, **characterised in that** at each outer end (17) of the cover lens (5) there is a cover lens tightener (9) with a recess (14) and at each outer end of the frame (2) a tightening hook (8) is formed, wherein the cover lens tightener (9) is made at least in sections from an elastically stretchable material, wherein the spacing of the tightening hooks (8) along the lens (4) is greater than the spacing of the rear ends of the recesses (14), so that the rear ends of the recesses (14) can be latched behind the tightening hooks (8) only by the elastic stretching of the cover lens tightener (9) over the rear ends of the tightening hooks (8).

2. Goggles according to claim 1, **characterised in that** adjoining the rear end of the recesses (14) handling attachments (16) are provided, which bear in the inserted tightened state on the rear end of the tightening hooks (8).

3. Goggles according to claim 1, **characterised in that** the cover lens tighteners (1) comprise a base body (10) made of an elastic material, which is provided with a peripheral groove (11), so that the base body (10) can be inserted into a recess of the cover lens (5), such that the edge of the recess of the cover lens (5) comes to lie in the groove (11) of the base body (10) and the cover lens tightener (8) is fixed in this way on the cover lens (5).

## Revendications

1. Lunettes, en particulier lunettes de sport ou lunettes de ski, comportant une monture et un disque disposé sur la monture, ainsi qu'un écran de recouvrement pouvant être fixé de manière amovible sur le disque, **caractérisées en ce qu'**à chaque extrémité externe (17) de l'écran de recouvrement (5) est disposé un élément tendeur de l'écran de recouvrement (9) comprenant un évidement (14) et qu'à chaque extrémité externe de la monture (2), il y a un crochet de serrage (8), l'élément tendeur de l'écran de recouvrement (9) étant au moins partiellement constitué d'un matériau élastique extensible, la distance entre les crochets de serrage (8) tout le long du disque (4) étant supérieure à la distance entre les parties d'extrémité arrières des évidements (14) de sorte que les parties arrières des évidements (14) ne peuvent être encliquetées derrière les crochets de serrage (8), par-dessus les extrémités arrières des crochets de serrage (8), que lors d'une tension élastique des éléments tendeurs de l'écran de recouvrement (9).

2. Lunettes selon la revendication 1 **caractérisées en ce que** des embouts de maniements (16) sont prévus à l'extrémité arrière des évidements (14), embouts qui se plaquent sur l'extrémité arrière des crochets de serrage (8) en position d'emploi sous tension.

3. Lunettes selon la revendication 1 **caractérisées en ce que** les éléments tendeurs de l'écran de recouvrement (9) présentent un corps de base (10) dans un matériau élastique, qui est muni d'une rainure (11) circonférentielle, de sorte que le corps de base (10) peut être logé dans un des évidements de l'écran de recouvrement (5) de telle manière que le bord de l'évidement de l'écran de recouvrement (5) arrive à se loger dans la rainure (11) du corps de base (10) et que l'élément tendeur de l'écran de recouvrement (8) est ainsi fixé sur l'écran de recouvrement (5).
